# EUROPEAN PATENT SPECIFICATION

(11) **EP 2 151 182 B1**
(45) Date of publication and mention of the grant of the patent: **30.05.2012**
(21) Application number: 09009570.4
(22) Date of filing: 23.07.2009
(51) Int. Cl.: A61B 1/00, A61B 1/005, A61B 19/00, A61B 17/00

(54) **Active medical apparatus system**
Aktives medizinisches Vorrichtungssystem
Système d'appareil médical actif

(30) Priority: 06.08.2008 JP 2008203373
(43) Date of publication of application: 10.02.2010
(73) Proprietor: Olympus Medical Systems Corp., Tokyo 151-0072 (JP)
(72) Inventor: Yoshie, Michifumi, Tokyo 151-0072 (JP)
(74) Representative: von Hellfeld, Axel

(56) References cited:
- EP-A1- 1 908 389
- WO-A1-2007/080783
- WO-A1-2008/070556
- US-A1- 2005 119 527
- US-A1- 2006 069 310

## Description

### BACKGROUND OF THE INVENTION

### 1. Field of the Invention

The present invention relates to an active medical apparatus system, in which an active medical apparatus is driven on the basis of an instruction signal from an instruction input section. More particularly, the active medical apparatus system relates to an electric bending endoscope which has a flexible bending section and which is electrically driven by the instruction signal of the instruction input section, an active treatment instrument which has a joint that can be curved or bent, or an active overtube which has a bendable bending section.

### 2. Description of the Related Art

In recent years, an endoscope has been widely used in a medical field and an industrial field. In the medical field, a treatment instrument is also widely used in combination with an endoscope.

As the endoscope used as such medical apparatus, there are practically used an endoscope in which a bending section is bent by a manual operation, and a so-called electric bending endoscope in which the bending section is electrically driven and bent by a driving force of a motor.

Also, as the treatment instrument, there is known a so-called active treatment instrument in which a curvable or bendable joint provided on the distal end side of the treatment instrument is electrically driven by a driving force of a motor.

For example, an electric bending endoscope is disclosed in Japanese Patent Application Laid-Open Publication No. 2007-185355.

In the electric bending endoscope disclosed in Japanese Patent Application Laid-Open Publication No. 2007-185355, a control section rotationally drives a motor as a driving section via a motor driver on the basis of an instruction inputted by an operation of a joystick (instruction operation section) by an operator, so that the rotating motor pulls a bending wire so as to thereby drive and bend a bending section provided in an insertion section of the endoscope. Further, the electric bending endoscope has a so-called force sense feedback function in which the bending state of the bending section is detected by a bending state detecting section, and in which according to the bending state, a reaction force is generated by a servo motor in the tilting direction of the joystick.

However, as described in Japanese Patent Application Laid-Open Publication No. 2007-185355, in the state where the force sense feedback is made to function, when the operator releases the hand from the joystick, the joystick is abruptly moved in the direction in which the force sense feedback is applied (that is, in the direction in which the joystick is moved by the servo motor), and the bending section is also abruptly moved according to the abrupt movement of the joystick. As a result, an operation which is not intended by the operator is performed.

For this reason, when such unintended operation can be effectively suppressed, it is possible to provide a desirable system which is conveniently used or efficiently operated by the operator.

In other words, in the system having the force sense feedback function, there is desired a system which is capable of, in such a case where the operator releases the hand from the instruction input section, suppressing the electric bending endoscope, the active treatment instrument, the active overtube active medical apparatus, and the like, from being moved by the feedback in a direction which is not intended by the operator.

WO/2008/070556 discloses a damper that sets a minimum and/or maximum threshold of force and velocity a user may apply on, for example, a joystick, such that movements below or above these thresholds will not move the endoscope and generally discloses that a damper may smooth out jerky or inconsistent joystick movements.

WO/2008/070556 further discloses a position or force sensor" that measures the amount of force applied by a user and force feedback that represents a force acting on an endoscope such that when an endoscope encounters an anatomical structure the user can feel the tool making contact with the structure via a feedback force applied to a joystick.

WO/2008/070556, however, lacks any mechanism of preventing a movement of a surgical tool in the feedback direction so to prevent unwanted movements of a surgical tool.

US Patent Pub. No. 2005/0119527 discloses a variable braking function, wherein a user can set a variable braking threshold between 0 and the maximum torque that can be supplied by servo motors. A servo control unit checks whether the torque on one of more servo motors surpasses this threshold. If the threshold has been surpassed, the endoscope automatically moves to a configuration that lowers the torque below the threshold and results in also lowering the amount of force an endoscope is applying to an anatomical structure.

US Patent Pub. No. 2005/0119527, however, lacks any mechanism of preventing a movement of a surgical tool in the feedback direction so to prevent unwanted movements of a surgical tool.

EP 1 908 389 discloses point-locking an arbitrary link member among a plurality of link members constituting a bending portion of an insertion portion of an endoscope, and attitude controlling the other link members so that it is possible to insert the bending portion of the insertion portion in a shape conforming to the interior shape of a tube cavity. As a result, improved insertability of the insertion portion is achieved.

EP 1 908 389 further discloses a load detecting section that detects a force created from the endoscope coming into contact with an anatomical structure, and outputs the detection result.

EP 1 908 389, however, lacks any mechanism of preventing a movement of a surgical tool in the feedback direction so to prevent unwanted movements of a surgical tool.

### SUMMARY OF THE INVENTION

The present invention has been made in view of the above-described circumstances. An object of the present invention is to provide, in the active medical apparatus system in which force information is fed back, a system which is capable of, when movement in an amount of a predetermined threshold value or more is generated in a direction which is not intended by the operator, suppressing the movement.

An active medical apparatus system according to the present invention includes:
an active medical apparatus having at least one rotatable joint;
an active medical apparatus driving section which electrically drives the active medical apparatus;
an instruction input section with which an operator to perform instruction input to drive the active medical apparatus;
an instruction input section driving section to which information of a force acting on the active medical apparatus is fed back and inputted, and which electrically drives the instruction input section according to the information of the force;
a determining section which determines whether or not a movement of the instruction input section in an acting direction of a driving force driving the instruction input section is generated in an amount of a predetermined threshold value or more; and
a control section which, in a case where the movement of the instruction input section is generated in the amount of the threshold value or more, suppresses at least one of the driving by the instruction input section driving section and the driving by the active medical device driving section.

### BRIEF DESCRIPTION OF THE DRAWINGS

Fig. 1 is a perspective view showing an appearance of a treatment instrument system of an embodiment 1 according to the present invention;
Fig. 2 is a figure showing an internal configuration of the treatment instrument system of embodiment 1;
Fig. 3 is a block diagram showing an exemplary configuration of a control system used at a normal time in embodiment 1;
Fig. 4 is a flow chart showing a processing procedure of embodiment 1;
Fig. 5 is a block diagram showing an exemplary configuration of the control system used at a specific time in embodiment 1;
Fig. 6 is a figure explaining an operation in embodiment 1;
Fig. 7 is a block diagram showing a force feedback type bilateral control system as another exemplary configuration of the control system at the normal time;
Fig. 8 is a block diagram showing a symmetric type bilateral control system as another exemplary configuration of the control system at the normal time;
Fig. 9 is a block diagram showing a parallel type bilateral control system as another exemplary configuration of the control system at the normal time;
Fig. 10 is a perspective view showing an appearance of a treatment instrument system of embodiment 2 according to the present invention;
Fig. 11 is a perspective view showing an appearance of a treatment instrument system as a modification of the embodiment 2;
Fig. 12 is a perspective view showing a configuration of a treatment section;
Fig. 13 is a figure showing a configuration of an endoscope system of embodiment 3 according to the present invention;
Fig. 14 is a block diagram showing an exemplary configuration of a control system used at the normal time in embodiment 3; and
Fig. 15 is a flow chart showing a processing procedure of embodiment 3.

### DETAILED DESCRIPTION OF THE PREFERRED EMBODIMENTS

In the following, embodiments according to the present invention will be described with reference to the accompanying drawings. Note that the term 'suppressing' based on determination, which term is used in the specification, means that the operation of the instruction input section by the instruction input driving section, as will be described below, and the operation of the active medical apparatus, which follows the operation of the instruction input section, are regulated so that the movement due to the operations is reduced or stopped so as to be limited within a movement amount intended by the operator.

### (Embodiment 1)

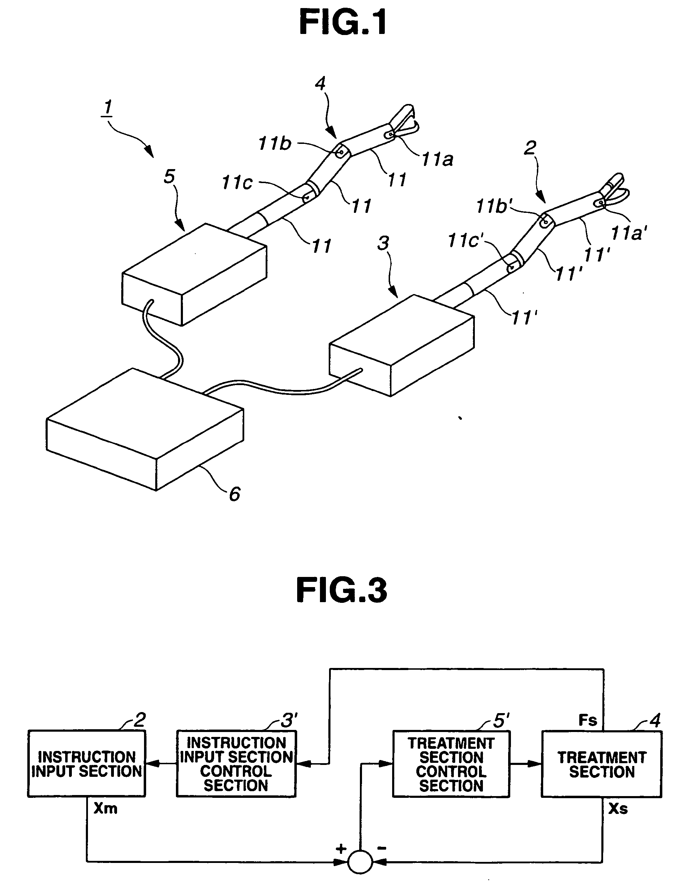

The present embodiment as shown in Fig. 1 to Fig. 9 is configured by a treatment instrument having an active mechanism.

The treatment instrument system 1 of embodiment 1 according to the present invention, as shown in Fig. 1, includes: an instruction input section 2 which has a movable section and which is used by an operator, such as a surgeon, to perform an instruction input (or instruction operation); an instruction input section driving section 3 which electrically drives the instruction input section 2; a treatment section 4 in which there is formed an active medical apparatus having a movable section for performing treatment; a treatment section driving section 5 which electrically drives the treatment section 4 according to the instruction input by the instruction input section 2; and a control section 6 which performs drive control of the instruction input section driving section 3 and the treatment section driving section 5.

In the embodiment shown in Fig. 1, the instruction input section 2 is configured to have an instruction input active medical apparatus whose shape is substantially similar to or analogous to the shape of an active medical apparatus which configures at least a movable section in the treatment section 4.

Specifically, the treatment section 4 has, as a movable section for performing treatment, an active medical apparatus structure in which circular pipe-shaped joint pieces (or arms) 11, 11 and 11 are provided so as to be rotatable around joint shafts 11 a, 11b and 11c, respectively.

Similarly, the instruction input section 2, with which the operator performs an instruction operation to the treatment section 4, also has circular pipe-shaped joint pieces 11', 11' and 11' which serve as movable sections for performing the instruction input operation, and which are respectively configured so as to be rotatable around the joint shafts 11a', 11b' and 11c', and the like.

When the operator grasps the side of the instruction input section 2 and performs the instruction input operation, specifically, an operation of the respective joint pieces by rotating the joint shafts 11a', 11b' and 11c', then the joint shafts 11a, 11b and 11c on the side of the treatment section 4 are also driven by the control of the control section 6 so as to effect the position and attitude of the treatment section 4, which correspond to the position and attitude of the instruction input section 2.

For this reason, when the instruction input section 2 is regarded as a master for an active medical apparatus (master), the treatment section 4 can be regarded as the active medical apparatus (slave).

Further, when the master is grasped and operated by the operator so as to bring the active medical apparatus into desired position and attitude states, the active medical apparatus can be set to the same position and attitude states as those of the master in such a manner that the active medical apparatus is driven by the treatment section driving section 5 under the control of the control section 6 so as to follow the position and attitude of the master.

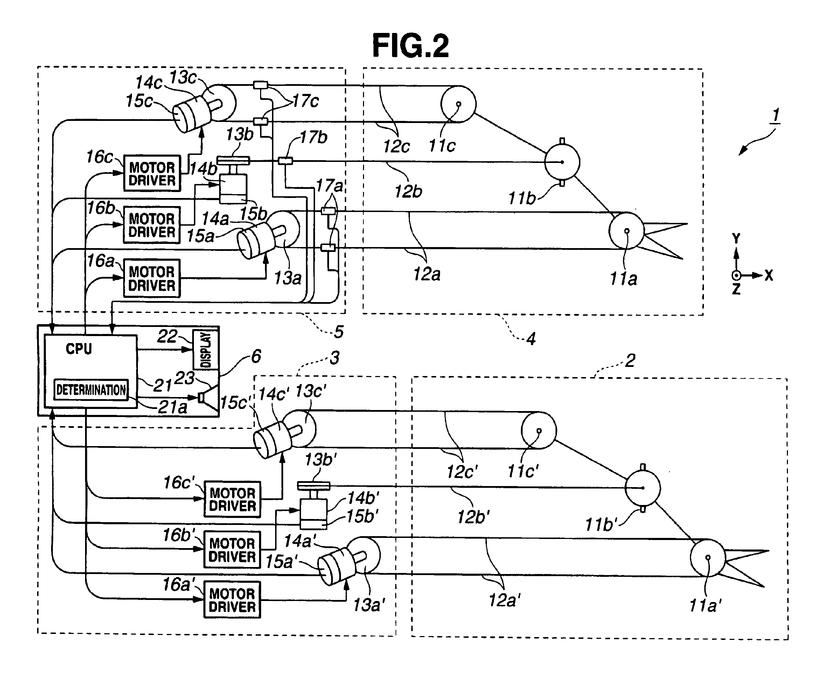

Fig. 2 shows an internal configuration of the treatment instrument system 1. Note that joint shafts (rotary shaft) 11a to 11c, 11a' to 11c', and the like, are schematically shown in Fig. 2.

As described above, the treatment section 4 and the instruction input section 2 have the plurality of j oint shafts 11a to 11c and the plurality of joint shafts 11 a' to 11c', respectively.

Specifically, as shown in Fig. 2, when the paper surface is set as the X-Y plane, the most distal end joint shaft (rotary shaft) 11a is set in the Z-axis direction vertical to the paper surface, and a next joint shaft 11b is set in the Y-axis direction in the paper surface. Further, a next joint shaft 11c is set in the Z-axis direction vertical to the paper surface.

Note that the joint shafts 11a' to 11c' on the other side are set similarly to the case of the joint shafts 11a to 11c.

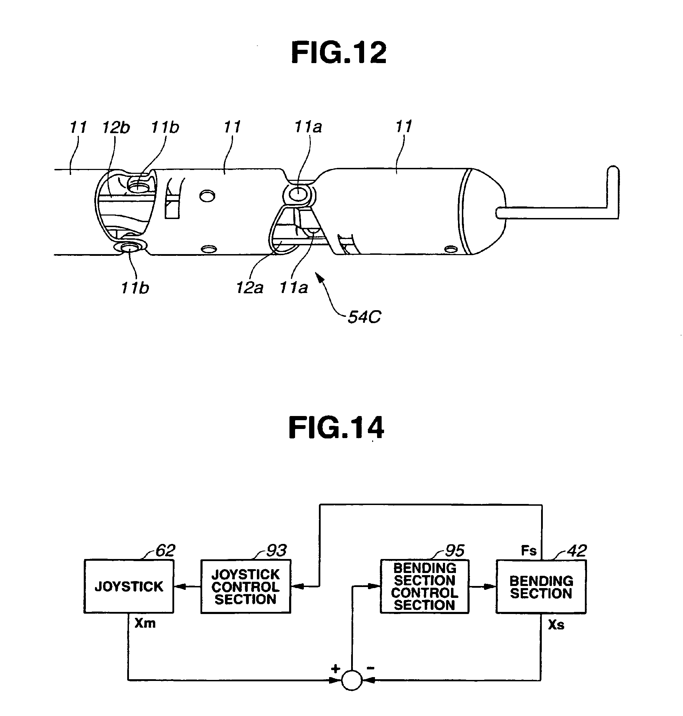

Further, wires 12a, 12a; 12b, 12b; 12c, 12c, which transmit the force for rotationally driving the respective joint shafts 11a to 11c are inserted in (the joint pieces 11 of) the treatment section 4, (see Fig. 12 showing a similar specific configuration as will be described below).

The distal ends of the wires 12i and 12i (i = a to c) are fixed around the joint shaft 11i, while the rear ends of the wires 12i and 12i are fixed by being hooked or wound around a pulley 13i in the treatment section driving section 5.

Further, the pulley 13i is attached to a rotary shaft of a motor 14i as a driving section (or power section). To the rotary shaft of the motor 14i, there is attached, for example, a rotary encoder (hereinafter abbreviated simply as encoder) 15i serving as a position sensor for detecting an angle of rotation (rotation angle) of the rotary shaft. Note that a gear (not shown) is usually attached to the motor 14i.

The each motor 14i is rotated by receiving a drive signal from each motor driver 16i.

Further, on the rear end side of the wires 12i and 12i, there are attached tension sensors 17i and 17i which detect the force acting on the respective wires 12i and 12i, in other words, the force (torque) for rotating the joint piece around the joint shaft 11i. Note that the positions at which the tension sensors 17i and 17i are provided are not limited to the vicinity on the rear end side of the wires 12i and 12i, and there may be selected arbitrary positions at which the tension sensors 17i and 17i can be attached. Further, the sensor attached to the wire is not limited to the tension sensor, and any sensor, such as a pressure-sensitive sensor and a distortion sensor, can be suitably used as long as the sensor is capable of directly or indirectly detecting the tension.

Further, the force information based on the signal detected by the tension sensor 17i and the position information based on the signal detected by the encoder 15i are inputted into a CPU 21 which configures the control section 6. The CPU 21 controls the rotation of the motor 14i via the motor driver 16i.

Further, the configuration of the instruction input section 2 is the same as that of the treatment section 4, and hence the components of the instruction input section 2 are represented by attaching the mark' to the components of the treatment section 4.

Further, the configuration of the instruction input section driving section 3 is the same as the configuration of the treatment section driving section 5 except that the tension sensors 17i provided in the treatment section driving section 5 are not provided. Thus, the components of the instruction input section driving section 3 are represented by attaching the mark' to the components of the treatment section driving section 5.

To the CPU 21 which configures the control section 6, there are inputted, as position information, signals detected by encoders 15a' to 15c' which can detect the instruction input operation of the instruction input section 2 performed by the operator.

That is, the rotation directions and angles of rotation (defined as rotation angles) of the joint shafts 11a' to 11c' which are operated by the operator (and which configure the master) are detected by the encoders 15a' to 15c'. The encoder output signals are inputted into the CPU 21 as detection signals. At this time, the CPU 21 compares the inputted encoder output signals with the detection signals from the encoders 15a to 15c that respectively detect the rotation angles of the joint shafts 11a to 11c which are located on the side of the treatment section 4 (that is, which configure the active medical apparatus).

Then, the CPU 21 generates detection signals of difference values obtained by respectively subtracting the detection signals from the encoders 15a to 15c from the detection signals from the encoders 15a' to 15c', and sets the obtained difference values as instruction values for driving the treatment section 4, so as to rotate the joint shafts 11a to 11c via the motor drivers 16a to 16c.

Thereby, the position and attitude of the treatment section 4 are controlled so as to follow the position and attitude of the instruction input section 2.

In the present embodiment, the force applied to effect the rotation around each of the joint shafts 11a to 11c is detected by each of the tension sensors 17a to 17c, and the detection signals are also inputted as force information into the CPU 21.

Then, the CPU 21 feeds back the detection signals so as to drive the joint shafts 11a' to 11c' via motor drivers 16a' to 16c'. In this way, force information feedback means is formed so that the forces acting on each of the joint shafts 11a to 11c are reflected on each side of the joint shafts 11a' to 11c' Note that it may also be configured such that in the case of performing the feedback, a gain, and the like, as control parameters, can be variably set beforehand. Note that as an specific example to suppress the driving of the instruction input section driving section 3 and the treatment section driving section 5 on the basis of a result of determination by a determining section 21 a, as will be described below, a method of changing the control mode or a method of changing the control parameter value may also be adopted in addition to the method of stopping or reducing the driving of the instruction input section driving section 3 and the treatment section driving section 5.

When the force information is fed back in this way, the force acting on the treatment section 4 is reproduced in the instruction input section 2 to the operator who operates the instruction input section 2. Thereby, the operability is improved so that the treatment can be performed by the treatment section 4 in a state close to the environment in which the treatment is directly performed by the instruction input section 2.

Note that in the present embodiment, there are provided the position sensor which detects the position information on the side of the treatment section 4, and the force sensor which detects the force information on the side of the treatment section 4. However, when the force information can be calculated by the position sensor, the present embodiment may also be configured by providing only the position sensor.

In the present embodiment, in order to further improve the operability, the CPU 21 which configures the control section 6 monitors always (as a specific example, for each substantially fixed period) the force information given to the instruction input section 2. More specifically, the CPU 21 monitors the directions of the DC current instruction values (directions of DC currents) supplied to the motor drivers 16a' to 16c', and the amount of movement of the instruction input section 2 at that time (specifically, the amount of movement per unit time, which is obtained from the detection signals of the encoders 15a' to 15c').

By monitoring in this way, the CPU 21 is able to have a function of the determining section 21a which determines whether or not the direction of the force applied to the instruction input section 2 coincides with the moving direction of the instruction input section 2, and whether or not the amount of movement of the instruction input section 2 exceeds a threshold value.

When it is determined by the determining section 21 a that the acting direction of the driving force coincides with the direction of the movement amount of the instruction input section 2, and that the amount of movement exceeds the threshold value, the CPU 21 changes, for example, the control state of the control system.

Note that when such determination is performed, the determination, in the present embodiment, is independently performed for each of the plurality of joint shafts 11a' to 11c'.

That is, the determination is performed similarly to the case of a treatment instrument having only one joint shaft, and thereby the determination can be applied similarly to the case of the plurality of joint shafts.

There are several specific examples of changing the control state. Basically, however, the control state of the control system is changed to a control state to suppress the movement of the instruction input section 2 at the time when it is determined by the determining section 21 a that the movement amount of the instruction input section 2 exceeds the threshold value.

Further, in the case where the determination that the movement amount exceeds the threshold value is outputted by the determining section 21 a, the CPU 21 displays the state of the movement amount exceeding the threshold value by a display section 22 serving as a presentation section for presenting the determination information as shown in Fig. 2, and also performs audio notification by using a loudspeaker 23.

Further, when obtaining the determination that the movement amount exceeds the threshold value, the CPU 21 starts a timer (not shown), so as to restore the control state to the normal control state after the lapse of a fixed time.

Fig. 3 is a block diagram of a force reflecting type bilateral control system as an exemplary configuration of a control mode of the control system, which is used (at the normal time) in the present embodiment.

Note that an instruction input section control section 3' in Fig. 3 is shown by including the instruction input section driving section 3 in Fig. 2 and a part of the mechanism of the control section 6, which directly controls the instruction input section driving section 3. Similarly, a treatment section control section 5' is also shown by including the treatment section driving section 5 in Fig. 2 and a part of the mechanism of the control section 6, which directly controls the treatment section driving section 5.

Further, position information determined by rotation angles of the plurality of joint shafts 11a' to 11c' in Fig. 2 is generally referred to as position information Xm of the instruction input section 2 in Fig. 3, and position information determined by rotation angles of the plurality of joint shafts 11a to 11c in Fig. 2 is generally referred to as position information Xs of the treatment section 4 in Fig. 3. Similarly, information of the forces acting on the plurality of joint shafts 11a' to 11c' is also represented by force information Fs of the treatment section 4 in Fig. 3. In the force reflecting type bilateral control shown in Fig. 3, the position information Xm generated by the input instruction given to the instruction input section 2 is subtracted by the position information Xs of the treatment section 4, and the subtracted position information (Xm - Xs) is sent to the treatment section control section 5'. The treatment section control section 5' performs position control of the treatment section 4 on the basis of subtracted position information (Xm - Xs).

Further, the force information Fs applied to the treatment section 4 is sent to the instruction input section control section 3'. The instruction input section control section 3' performs force control (driving) of the instruction input section 2 on the basis of the force information Fs.

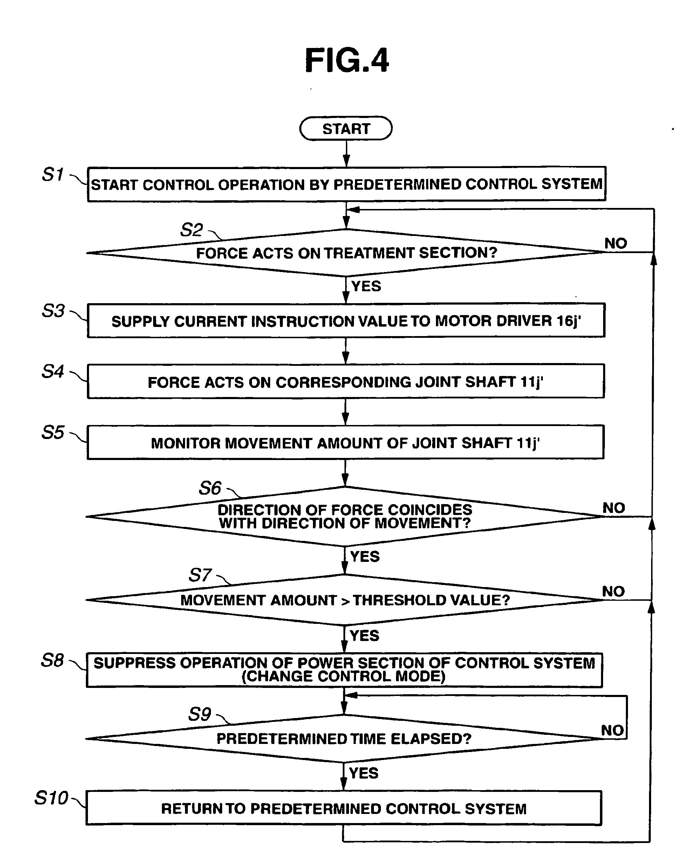

Next, a processing procedure including the determination by (the determining section 21 a of) the CPU 21, according to the present embodiment, will be described with reference to Fig. 4.

When the power source of the treatment instrument system 1 shown in Fig. 2 is turned on, the treatment instrument system 1 starts a control operation in (a control mode of) the predetermined control system, as shown in step S 1. Specifically, the CPU 21 starts a control operation in the control mode based on the force reflecting type bilateral control system shown in Fig. 3, that is, in the force reflecting type bilateral control mode.

In next step S2, the CPU 21 is set in the processing state to wait for the force to be made to act around the joint shafts 11a to 11c of the treatment section 4 (to wait for detection signals of the tension sensors 17a to 17c). Then, when detecting the action of the force, the CPU 21 supplies current instruction values corresponding to the force to the motor driver 16j' as shown in step S3.

Here, the motor driver 16j' receives the current instruction value corresponding to the signal detected by the tension sensor 17i of the tension sensors 17a to 17c. Further, the current instruction value includes the direction in which a motor 14j' is rotated.

Note that when the action of the force around the plurality of joint shafts is detected, the control operation shown in Fig. 4 is independently performed to each of the joint shafts.

Then as shown in step S4, the motor 14j' driven by the motor driver 16j' supplies the force to effect the rotation around the corresponding joint shaft 11j'.

Further, as shown in step S5, the CPU 21 monitors the rotation angle of the joint shaft 11j' of the instruction input section 2 on the basis of the detection signal of the encoder 15j'.

Then, as shown in step S6, the CPU 21 determines whether or not the direction of the force (driving force) to rotate the joint shaft 11j' coincides with the direction of movement (detected in the preceding step S4). When the directions are not coincident with each other, the CPU 21 returns to step S2.

On the other hand, when determining that the directions are coincident with each other, the CPU 21 determines, in next step S7, whether or not the amount of movement (movement amount) exceeds a predetermined threshold value. In other words, the CPU 21 determines whether or not movement in the acting direction of the driving force driving the instruction input section 2 is generated in an amount exceeding the predetermined threshold value.

When the movement amount does not exceed the threshold value, the CPU 21 returns to step 2. On the contrary, when the movement amount exceeds the threshold value, the CPU 21 suppresses, in step S8, the operation of the power section (driving section) in the control system, or changes the control mode to the other control mode so as to suppress the operation of the power section.

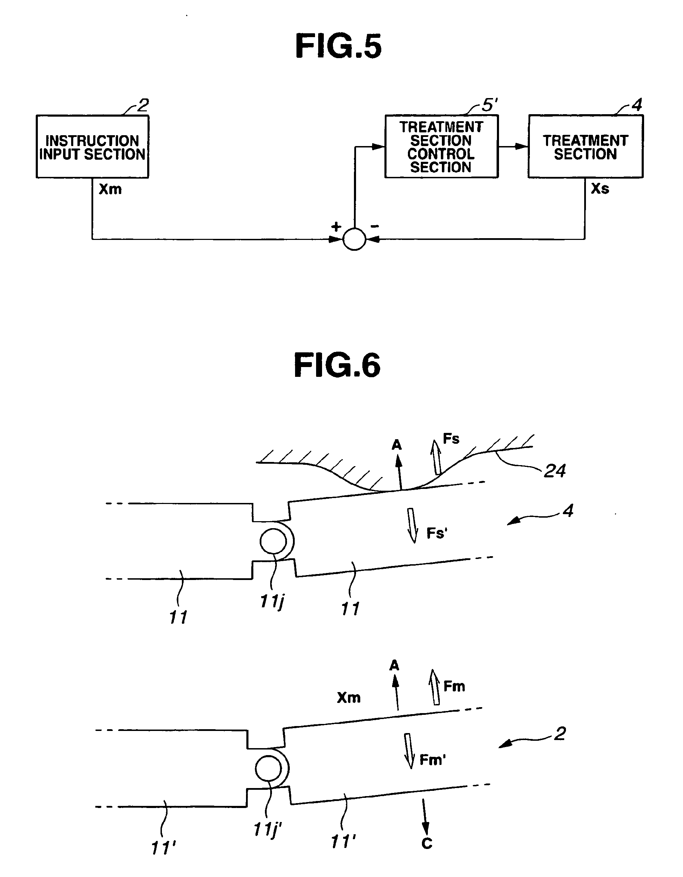

Specifically, the CPU 21 stops the operation of both the power sections of the motors 14j' and 14j, or stops the operation of at least one of the power sections of the motors 14j' and 14j. Alternatively, in the case where the control mode of the force reflecting type bilateral system as shown in Fig. 3 is adopted in the control system before the determination, the CPU 21 may change the control mode of the force reflecting type bilateral system to, for example, a control mode of an unilateral control system, as a control mode at the specific time as shown in Fig. 5, that is, an unilateral control mode.

In addition, for example, the CPU 21 may change control parameters without changing the control mode. For example, in the state where the force reflecting type bilateral control mode shown in Fig. 3 is maintained, the CPU 21 may change the control parameter used to drive the instruction input section 2 from the instruction input section control section 3', and the control parameter used to drive the treatment section 4 from the treatment section control section 5'.

The change of the parameter in this case may be performed so as to suppress (stop or reduce) at least the driving of the instruction input section 2 and the driving of the treatment section 4.

In the control mode of the unilateral control system shown in Fig. 5, the instruction input section 2 is separated from the instruction input section control section 3'. The instruction input section 2 is not driven by the instruction input section driving section 3'. That is, in the control mode of the unilateral control system, the state of the control mode is set such that the instruction input section 2 is not driven even when force is made to act on the treatment section 4, so as to be fed back as the force information to the instruction input section control section 3'.

In other words, in the control mode of the unilateral control system, only the position control of the treatment section 4 is performed by the input instruction given to the instruction input section 2, and feedback means of the force information is not provided.

For this reason, when the control mode is changed to the control mode of the unilateral control system according to the determination result (that the movement amount exceeds the threshold value), then unintended movement of the instruction input section 2 and the treatment section 4 can be cancelled (and thereby suppressed) as long as the operator does not actually perform an input instruction to the instruction input section 2.

Further, in this case, the determination result in step S7 or the information in step S8 is notified to the operator by the display section 22, and the like. Further, in next step S9, the CPU 21 is set in the state to wait for the elapse of the predetermined time on the basis of the timer started by the determination result.

When the predetermined time elapses, the CPU 21 performs, as shown in step S10, processing to restore the control state to the initial state (in other words, to cancel the suppression) and returns to the processing in step S2.

The operation in Fig. 4 will be supplementarily explained with reference to Fig. 6. Note that in Fig. 6, the right side of the treatment section 4 and the instruction input section 2 is the distal end side. Further, there is assumed a case where the operator (surgeon) inserts the treatment section 4 into a body, and performs treatment with the treatment section 4 by grasping with the fingers the instruction input section 2 located outside the body.

As described above, when the operator performs an operation to move the instruction input section 2 around the joint shaft 11j', for example, in the arrow A direction by grasping the instruction input section 2 with the fingers, then the treatment section 4 is also moved in the same direction A around the joint shaft 11j so as to follow the position information Xm.

In this case, when the force applied to effect the movement around the joint shaft 11j is the force information Fs, the force information Fs is fed back via the control section 6, so as to be applied as force information Fm to the joint shaft 11j'

When the side of the treatment section 4 is brought into contact with a wall surface 24 of, for example, an internal organ, or the like, within the body, and when the wall surface 24 hinders the rotation of the joint shaft 11j to generate reaction force Fs', the reaction force Fs' is also fed back, so as to be applied to the joint shaft 11j' as reaction force information Fm'.

In the normal operation state, by the force feed back, the operator is able to grasp (recognize), with the fingers (hand) grasping the instruction input section 2, the force Fs and the reaction force Fs', which act on the joint shaft 11j on the side of the treatment section 4, as the force Fm and the reaction force Fm'. Thus, the operator is able to grasp the magnitude and direction of the force also in the state where the side of the treatment section 4 is brought into contact with the wall surface 24, and hence the operability can be improved.

However, in the state where the reaction force Fm' is applied, for example when the operator carelessly releases the hand from the instruction input section 2, the force Fm generated by the operation by the operator is eliminated.

For this reason, the side of the instruction input section 2 is rapidly moved by reaction force information Fm' in the direction of the reaction force Fm' (the direction is indicated by the arrow C). Thereby, the position information Xm of the movement is also applied, as the instruction input on the side of the instruction input section 2, to the side of the treatment section 4, so as to also cause the side of the treatment section 4 to be rapidly moved in the direction of the arrow C.

That is, there is caused a state where an unintended operation is performed. However, the present embodiment is configured such that the direction of the reaction force applied to the instruction input section 2 in this case and the movement of the instruction input section 2 in the direction are monitored, and that when such movement is generated and when the movement amount exceeds the threshold value, the unintended operation is suppressed in such a manner of stopping the operation of the power section (or power source).

According to the present embodiment configured to function in this way, an unintended operation can be effectively prevented from being generated, and the good operability can be maintained.

Further, such unintended operation is temporarily generated, and hence the operation state is returned to the normal operation state after the lapse of the predetermined time, so as to thereby enable the operator to continue the control operation in the normal state of good operability.

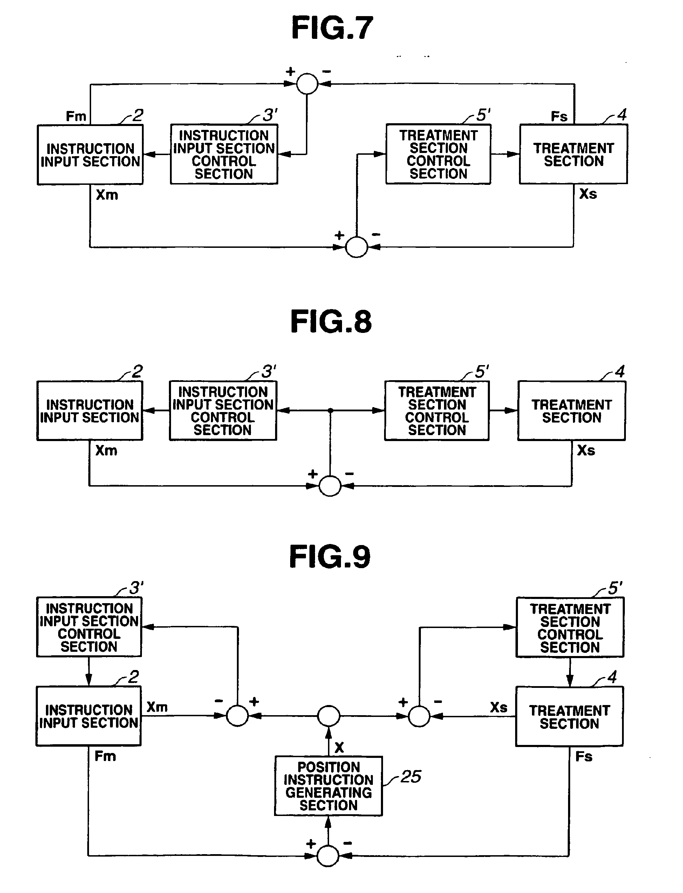

In the above description, the predetermined control system at the normal time is described as the force reflecting type bilateral control system shown in Fig. 3, but the present embodiment is not limited thereto. A force feedback type bilateral control system as shown in Fig. 7 may also be used.

The force feedback type bilateral control system is configured such that in the force reflecting type bilateral control system shown in Fig. 3, the force information Fm applied to the instruction input section 2 is further detected, and such that the force information (Fm - Fs) obtained by subtracting the force information of the treatment section 4 from the force information Fm is inputted into the instruction input section control section 3'.

In the force feedback type bilateral control system, power assist is performed in such a manner that in the force reflecting type bilateral control system, the force information Fm applied to the instruction input section 2 is returned to the instruction input section 2, as described above. Thus, the amount of operating force applied by the instruction input section 2 is reduced, so as to enable the operability to be improved.

Further, in the case where the force feedback type bilateral control system is used as the predetermined control system at the normal time, and where it is determined in step S7 in Fig. 4 that the movement amount exceeds the threshold value, the CPU 21 may, as described in step S8, suppress the operation of the power section of the control system or change the control mode of the control system.

Specifically, also in the case of the control mode of the force feedback type bilateral control system, the CPU 21 stops the operation of both the motors 14j and 14j', or, stops the operation of at least one of the motors. Alternatively, the CPU 21 may change the control mode to, for example, the control mode shown in Fig. 5.

Further, a symmetric type bilateral control system shown in Fig. 8 may also be adopted as the predetermined control system at the normal time. The symmetric type bilateral control system is a control method in which the position information of the instruction input section 2 is made to coincide with the position information of the treatment section 4, and has an advantage that the bilateral control can be realized without using the force sensor.

That is, in the symmetric type bilateral control system, the position information (Xm - Xs) obtained by subtracting the position information Xs of the treatment section 4 from the position information Xm of the instruction input section 2 is sent to the instruction input section control section 3' and the treatment section control section 5'. In the instruction input section control section 3' and the treatment section control section 5', the position control is respectively performed on the basis of the position information (Xm - Xs) so that the position of the instruction input section 2 and the position of the treatment section 4 are made to coincide with each other.

Also, in the case where the symmetric type bilateral control system is used, and where it is determined in step S7 in Fig. 4 that the movement amount exceeds the threshold value, the CPU 21 may, as described in step S8, suppress the operation of the power section of the control system or change the control mode of the control system.

Specifically, also in the case of the control mode based on the symmetric type bilateral control system, the CPU 21 stops the operation of both the motors 14j and 14j', or stops the operation of at least one of the motors. Alternatively, the CPU 21 may change the control mode.

It may also be configured such that a parallel type bilateral control system shown in Fig. 9 is adopted as the predetermined control system at the normal time. In the parallel type bilateral control system, force information (Fm - Fs) obtained by subtracting the force information Fs of the treatment section 4 from the force information Fm of the instruction input section 2 is inputted into a position instruction generating section 25, so that position information X as a position instruction is generated.

Further, in the parallel type bilateral control system, position information (X - Xm) obtained by subtracting the position information Xm of the instruction input section 2 from the position information X as the position instruction is sent to the instruction input section control section 3'. The instruction input section control section 3' controls the instruction input section 2 on the basis of the position information (X - Xm).

Further, position information (X - Xs) obtained by subtracting the position information Xs of the treatment section 4 from the position information X is sent to the treatment section control section 5'. The treatment section control section 5' controls the treatment section 4 on the basis of the position information (X - Xs).

Also, in this case, when it is determined in step S7 in Fig. 4 that the movement amount exceeds the threshold value, the CPU 21 may, as described in step S8, suppress the operation of the power section of the control system or change the control mode of the control system.

As described above, according to the present embodiment, the movement which is not intended by the operator can be suppressed, and the operability for the operator can be improved.

### (Embodiment 2)

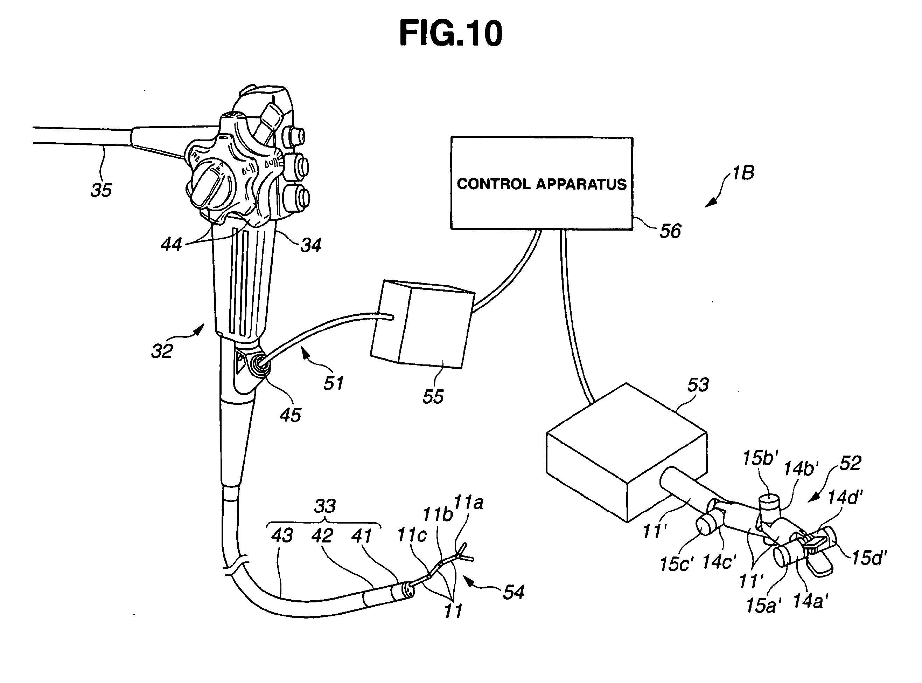

Next, there will be described embodiment 2 according to the present invention with reference to Fig. 10. Fig. 10 shows a treatment instrument system 1B of embodiment 2 according to the present invention. The treatment instrument system 1B is used in such a manner that a treatment instrument main body 51 is inserted into a channel provided in an insertion section 33 of an endoscope 32, which section is inserted into a body cavity.

The endoscope 32 includes the insertion section 33 which is inserted into the body cavity, an operation section 34 which is provided at the rear end of the insertion section 33, and a universal cable 35 which is extended from the operation section 34. A connector located at the end section (not shown) of the universal cable 35 is detachably connected to a light source apparatus and a signal processing apparatus.

The insertion section 33 includes a distal end section 41 in which an illumination window and an observation window are provided, a freely bendable bending section 42 which is provided at the rear end of the distal end section 41, and a long flexible section 43 which extends from the rear end of the bending section 42 to the operation section 34.

Further, a bending knob 44 for bending the bending section 42 is provided in the operation section 34. Further, a treatment instrument insertion port (hereinafter abbreviated simply as insertion port) 45 through which a treatment instrument is inserted, is provided near the front end of the operation section. The insertion port 45 communicates with the channel provided in the insertion section.

In the present embodiment, the thin and elongated treatment instrument main body 51 configuring the treatment instrument system 1B is inserted from the insertion port 45, and a treatment section at the distal end side of the treatment instrument main body 51 is made to project from the distal end opening of the channel so that treatment can be performed to a treatment object part.

The treatment instrument system 1B includes the treatment instrument main body (or treatment instrument) 51 at the distal end of which a treatment section 54 is provided, a motor box 55, as a power section, to which the rear end of the treatment instrument main body 51 is connected, an instruction input section 52 with which the operator performs an operation of instruction input, a driver box 53 in which motor drivers for the instruction input section 52 are housed, and a control apparatus 56 which controls the driver box 53 and the motor box 55.

The treatment section 54 similar to that shown in Fig. 1 is provided at the distal end of the thin and elongated flexible shaft section of the treatment instrument main body 51. However, in the present embodiment, the treatment section 54 is configured such that cup pieces are independently rotated around respective joint shafts (for the purpose of description, denoted by reference characters 11a and 11d).

Further, the respective joint shafts 11i are connected to a power section in the motor box 55 via the wires 12i. In the motor box 55, there are incorporated components of the treatment section driving section 5 including the motors 14a to 14c shown in Fig. 2. Also, there are additionally incorporated a motor 14d, an encoder 15d, and a tension sensor 17d in correspondence with that the joint shaft 11d is further added.

On the other hand, the instruction input section 52 is configured such that in the instruction input section 2 shown in Fig. 2, the motors 14a' to 14c' and the encoders 15a' to 15c' are provided in the instruction input section 52. Further, a motor 14d' and an encoder 15d' are additionally provided in correspondence with a joint shaft 11d' which is provided so as to correspond to the addition of the joint shaft 11d.

That is, the motors 14i' and the encoders 15i' are directly attached to the respective joint shafts which configure the instruction input section 52.

For this reason, in the present embodiment, the wires 12a' to 12c' in embodiment 1 are not used in the instruction input section 52. Further, the present embodiment is configured such that the motor drivers 16a' to 16c' shown in Fig. 2, and the motor driver 16d' are housed in the driver box 53.

Further, the control apparatus 56 has the same configuration as that of the control section 6, for example, shown in Fig. 2, and the control apparatus 56 is connected to the driver box 53 and the motor box 55 by cables, respectively.

Also, in the present embodiment, the instruction input section 52 is formed into a shape which simulates the treatment section 54, and is more specifically formed into a shape which is similar to and larger than the shape of the treatment section 54. However, in the exemplary configuration shown in Fig. 10, the shape of the instruction input section 52 is slightly different from the shape of the treatment section 54 in that the motor 14i' and encoder 15i' are projectingly provided.

In order to make the shape of the instruction input section 52 more similar to the shape of the treatment section 54, the present embodiment may also be configured such that, as in embodiment 1, the joint shafts of the instruction input section 52 are driven via wires similarly to the treatment section 54 without providing the motors 14i' and the encoders 15i' in the instruction input section 52.

In the present embodiment, treatment of a treatment object part, such as a lesion part in a body cavity, which part is observed by the endoscope 32, is performed by the treatment section 54, which is inserted in the channel of the endoscope 32 so as to project from the distal end opening of the channel.

In this case, by operating the instruction input section 52 having the shape similar to that of the treatment section 54, the surgeon as the operator is able to make the treatment section 54 follow the operational attitude of the instruction input section 52. For this reason, the treatment can be performed in a state of good operability.

Note that the control apparatus 56 performs the same control as that in the case of embodiment 1. For example, the control apparatus 56 performs the control shown in Fig. 4, and the like. According to the present embodiment, the operator is able to perform treatment to a lesion part, and the like, in a body cavity in a state of good operability.

Further, similarly to the case of embodiment 1, an unintended movement can be suppressed by the control operation shown in Fig. 4, and thereby the operability can be improved.

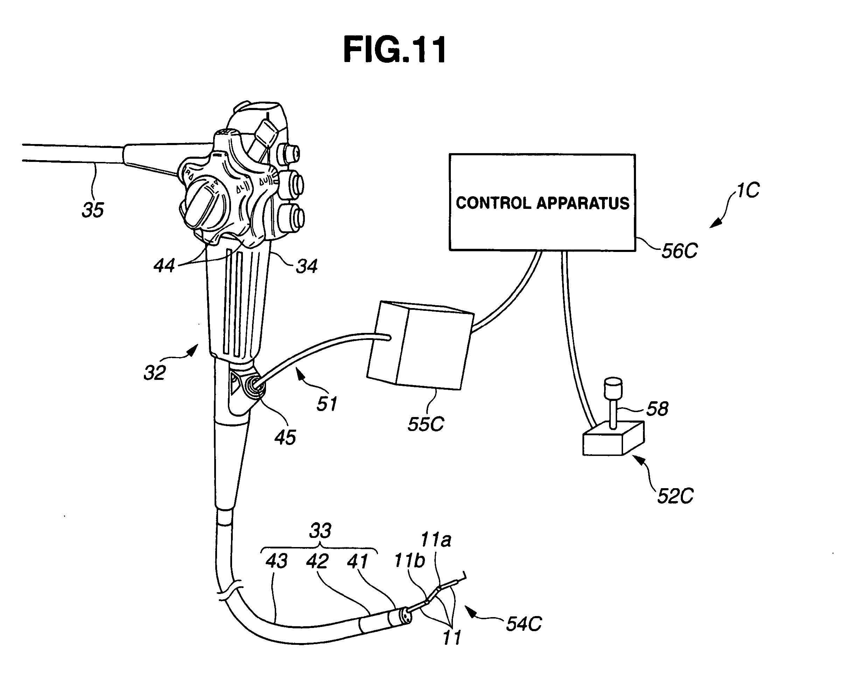

Fig. 11 shows a treatment instrument system 1C as a modification. In the treatment instrument system I C as the modification, instead of the treatment section 54 shown in Fig. 10, there is adopted, for example, a treatment section 54C having an L-shaped distal end treatment section having two joint shafts. Further, the treatment instrument system 1C is configured such that the master structure simulating the treatment section 54 is not used in the instruction input section 52, and that there is adopted a joystick apparatus 52C which is not similar to the treatment section 54C and which includes a joystick 58 as a stick that can be tilted in a plurality of different directions.

Further, in the instruction input operations performed by tilting the joystick 58 in the joystick apparatus 52C, the instruction input operation to tilt the joystick 58, for example, in the vertical direction corresponds to the rotation of the treatment section 54C around the joint shaft 11a, and the instruction input operation to tilt the joystick 58, for example, in the left and right direction corresponds to the rotation of the treatment section 54C around the joint shaft 11b. Note that in the exemplary configuration shown in Fig. 11, the function of the driver box 53 shown in Fig. 10 is incorporated in the joystick apparatus 52C or in a control apparatus 56C shown in Fig. 11.

Fig. 12 shows a specific configuration of the treatment section 54C. The joint pieces 11, 11 and 11, which are adjacent to each other, are connected to each other by joint shafts 11a and 11b, such as rivets, so as to be freely rotated in the directions perpendicular to each other. The wire 12a which is inserted into the joint piece 11 and which is used to rotate the joint shaft 11a in one direction (to substantially the lower side of the paper surface in Fig. 12) is fixed at a cutout section of the joint piece 11 in front of the joint shaft 11a. The wire 12a (not shown) which is used to rotate the joint shaft 11a in the reverse direction (to substantially the upper side of the paper surface in Fig. 12) is similarly fixed.

Similarly, the wire 12b which is used to rotate the joint shaft 11b in one direction (to substantially the upper side in the direction perpendicular to the paper surface in Fig. 12) is fixed at a cutout section in the joint piece 11 in front of the joint shaft 11b. The wire 12b (not shown) which is used to rotate the joint shaft 11b in the reverse direction is similarly fixed.

In the modification, for example, the numbers of the motors and the encoders in a motor box 55C are respectively reduced from four to two. Further, the number of the tension sensors is reduced from eight to four.

The numbers of the motors and the encoders on the side of the joystick apparatus 52C are the same as the numbers on the side of the motor box 55C.

The other configuration is the same as the configuration in embodiment 2. In the present modification, by the tilting operation of the joystick 58, the treatment section 54C can be controlled to be set in the attitude state corresponding to the tilting operation of the joystick 58.

The effect of the present modification is substantially the same as the effect of embodiment 2 or embodiment 1.

### (Embodiment 3)

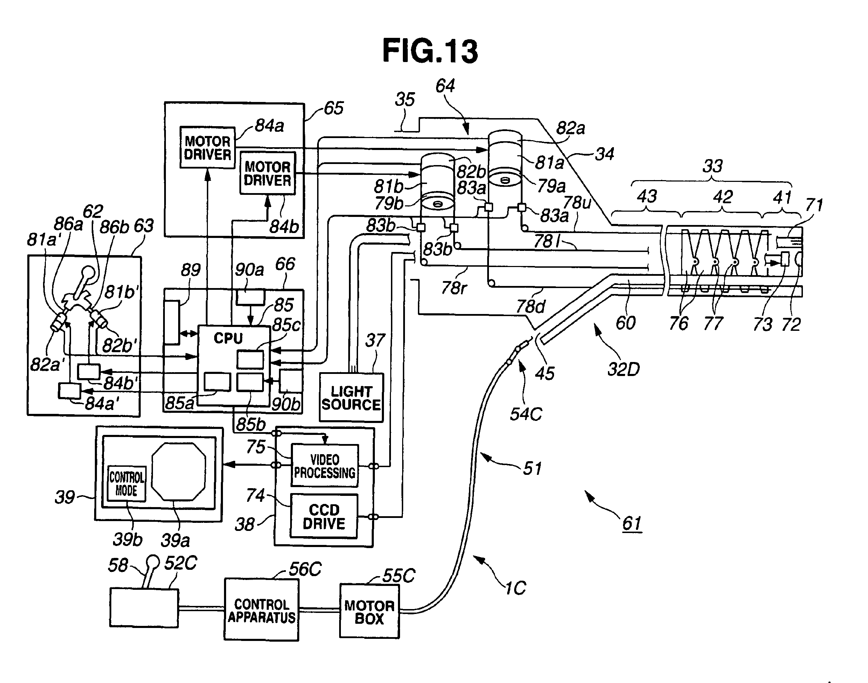

Next, there will be described embodiment 3 according to the present invention with reference to Fig. 13. Fig. 13 shows an endoscope system 61 of embodiment 3 according to the present invention. In embodiment 2, there is adopted the endoscope 32 in which the bending section 42 is manually bent by the operator performing the operation of manually rotating the bending knob 44.

On the other hand, the present embodiment includes an electric bending endoscope in which, when the operator performs an (instruction input) operation to tilt the joystick apparatus, the bending section 42 is electrically (actively) driven by using a power section (driving section).

The endoscope system 61 includes an electric bending endoscope (hereinafter simply referred to as endoscope) 32D, a light source apparatus 37 which supplies illumination light to the endoscope 32D, a video processor 38 as a signal processing apparatus which performs signal processing to an image pickup device of the endoscope 32D, and a display monitor 39 which displays a video signal outputted from the video processor 38.

Further, the endoscope 32D includes, similarly to the endoscope 32 shown in Fig. 10, the insertion section 33, the operation section 34, and the universal cable 35. The insertion section 33 includes the distal end section 41, the bending section 42, and the flexible section 43.

Further, the insertion port 45 is provided at the front end of the operation section 34. The insertion port 45 communicates with a channel 60 provided in the longitudinal direction of the insertion section 33. The operator is able to insert, from the insertion port 45, for example, the treatment instrument main body 51 which has the treatment section 54C and which forms the treatment instrument system 1C shown in Fig. 11.

Note that the operator is also able to perform treatment by inserting, into the insertion port 45, the treatment instrument system I B as described with reference to Fig. 10 instead of the treatment instrument system 1C. Further, the operator is also able to perform treatment by inserting, into the insertion port 45, a treatment instrument (not shown) without the power section.

Further, the endoscope system 61 includes a joystick apparatus 63 having a joystick 62 which is used to perform a bending instruction input operation, a motor box (or motor unit) 64 which is provided, for example, in the operation section 34, and which serves as the power section to drive and bend the bending section 42 forming the active medical apparatus in the present embodiment, a driver box 65 which drives motors as the power section in the motor box 64, and a control apparatus 66 which performs bending control of the bending section 42.

The illumination light from the light source apparatus 37 to which the connector provided at the end section of the universal cable 35 is detachably connected, is supplied to a light guide 71 of the endoscope 32D. The illumination light is emitted from the distal end surface of the light guide 71.

An objective lens 72 attached to an observation window forms an optical image of a subject, such as a diseased part, illuminated by the illumination light. A charge coupled device (hereinafter abbreviated as CCD) 73 is arranged at the position where the optical image of the subject is formed. The CCD 73 is connected to a CCD drive circuit 74 and a video processing circuit 75 in the video processor 38 via signal lines.

The CCD drive circuit 74 applies a CCD drive signal to the CCD 73, so as to enable an image pickup signal, which is subjected to photoelectric conversion by the CCD 73, to be outputted from the CCD 73. The image pickup signal outputted from the CCD 73 is converted into a video signal by signal processing performed by the video processing circuit 75. Then, the optical image formed in the CCD 73 is displayed as an endoscopic image in an endoscopic image display area 39a in the display surface of the display monitor 39 into which the video signal is inputted.

Further, in the bending section 42, a plurality of joint pieces or bending pieces 76, which are adjacent to each other, are rotatably connected to each other by rivets 77 serving as joint shafts (rotary shafts). Note that in Fig. 13, for the sake of simplicity, there are shown rivets 77 which can be freely rotated only in the direction perpendicular to the paper surface, but in practice, the bending pieces 76, which are adjacent to each other in the longitudinal direction, are connected to each other by the rivets 77 so as to be freely rotated alternately in the vertical direction and in the left and right direction.

Further, the distal ends of pairs of bending wires 78u, 78d; 781, 78r, which pairs are inserted in the insertion section 33 so as to be respectively arranged in the vertical direction and the right and left direction, are fixed to the most distal end bending piece 76 or the distal end section 41. The rear ends of the bending wires 78u and 78d and the rear ends of the bending wires 781 and 78r are respectively fixed in such a manner of being hooked to a vertical bending pulley 79a and a left and right bending pulley 79b.

The pulleys 79a and 79b are rotatably connected to rotary shafts of motors 81a and 81b as the power section, respectively. Encoders 82a and 82b are connected to the rotary shafts of the motors 81a and 81b, respectively. The encoders 82a and 82b detect the angles of rotation of the motors 81a and 81b, respectively.

Further, tension sensors 83a, 83a; 83b, 83b, which respectively detect tension acting on the bending wires 78u, 78d; 781, 78r, are respectively attached to the bending wires in front of the pulleys 79a and 79b. The motors 81a and 81 b, which drive and bend the bending section 42, are respectively connected to motor drivers 84a and 84b, so as to be rotated by receiving motor drive signals from the motor drivers 84a and 84b.

Further, the motor drivers 84a and 84b are connected to a CPU 85 which configures the control apparatus 66. The CPU 85 controls bending operations, such as a control operation of the motor drivers 84a and 84b.

Further, the detection signals of the encoders 82a and 82b and the detection signals of the tension sensors 83a to 83b are also inputted into the CPU 85.

Note that in the present embodiment, there are provided the encoders 82a and 82b as position sensors for detecting the position information on the side of the bending section 42, and the tension sensors 83a and 83b as force sensors for detecting the force information. However, when the force information can be calculated by the position sensors, the present embodiment may also be configured by providing only the position sensors.

Further, in the joystick apparatus 63, a rotary shaft of a motor 81a' as a power section is connected to a roller 86a which supports the proximal end section of the joystick 62 rotatably in the vertical direction. Further, an encoder 82a' is connected to the rotary shaft of the motor 81a'. The encoder 82a' detects a tilting angle (in other words, the angle of rotation of the motor 81 a') in the vertical direction of the joystick 62.

Similarly, a rotary shaft of a motor 81 b' as a power section is connected to a roller 86b which supports the proximal end section of the joystick 62 rotatably in the left and right direction. Further, an encoder 82b' is connected to the rotary shaft of the motor 81 b'. The encoder 82b' detects a tilting angle (in other words, the angle of rotation of the motor 81b') in the right and left direction of the joystick 62.

Further, the motors 81 a' and 81 b' are respectively connected to the motor drivers 84a' and 84b', and the operations of the motor drivers 84a' and 84b' are controlled by the CPU 85. The detection signals of the encoders 82a' and 82b' are inputted into the CPU 85.

The CPU 85 performs a bending control operation according to a program in a built-in flash memory 85a.

The bending control operation is based, for example, on the control mode of the force reflecting type bilateral control system as shown in Fig. 14, and is the same as the control mode of the force reflecting type bilateral control system shown in Fig. 3.

That is, the control contents shown in Fig. 14 are made to be the same as the control contents shown in Fig. 3 in such a way that the instruction input section 2 in Fig. 3 is replaced by the joystick 62, that the instruction input section control section 3' is similarly replaced by a joystick control section 93 as a control section which includes the power section of the joystick 62, that the treatment section 4 is replaced by the bending section 42, and that the treatment section control section 5' is replaced by a bending section control section 95 as a control section which includes the power section of the bending section 42. For this reason, the control mode in Fig. 14 is represented by using the position information Xm and Xs, and the force information Fs which are the same as those in Fig. 3.

Further, according to the program, the CPU 85 has a function of a determining section 85b which normally monitors, for each fixed period, whether or not there is generated a state where the direction of the driving force acting on the joystick 62 coincides with the moving (tilting) direction of the joystick 62, and which, when such state is generated, determines whether or not the amount of the movement (movement amount) exceeds a threshold value.

In other words, the determining section 85b determines whether or not the movement of the joystick is generated in an amount exceeding the predetermined threshold value in the acting direction of the driving force driving the joystick 62.

In the case where it is determined by the determining section 85b that the movement amount exceeds the threshold value, the state is displayed and presented to the operator or the like in the display section (as a presentation section of the determination information) of an operation panel 89 of the control apparatus 66.

Further, in the present embodiment, the CPU 85 sends the information about the state of bending control to the video processing circuit 75 of the video processor 38, so as to superpose the information on a video signal. Further, information of the operating control mode, and the like, is displayed in a bending control information display area 39b in the display monitor 39 together with a display of the following determination information.

As described above, in the case where it is determined by the determining section 85b that the movement amount exceeds the threshold value, the CPU 85 displays, in the bending control information display area 39b, the content that the movement amount has exceeded the threshold value, the content that the control mode is changed to a control mode different from the control mode at the normal time according to the determination result, and the like.

The information on the determination result by the determining section 85b, and the like, is displayed immediately near the endoscopic image, and thereby the operator is able to promptly know the information.

Note that in the present embodiment, the CPU 85, which performs the control operation of the control mode of the force reflecting type bilateral control shown in Fig. 14 at the normal time, has the control mode changing function to change the control mode to the other control mode on the basis of the determination result that the movement amount exceeds the threshold value, and also has the function of a control parameter setting section 85c which changes and sets control parameters for rotationally driving the motors 81 a, 81 b and 81 a', 81 b' that configure the power sections.

Among the control parameters after it is determined that the movement amount exceeds the threshold value, response speed control parameters are changed, for example, to reduce the response speed to be lower than the speed before the determination, and thereby an unintended operation is suppressed.

In addition, the control parameters related to the magnitude of the driving force may also be changed so as to reduce the driving force. It may be configured such that the function of the control parameter setting section 85c is also provided in embodiment 1, and the like.

Note that, in the present embodiment, there is provided in the control apparatus 66 a threshold setting section 90a used for variably setting the threshold value of the movement amount, which value is used for the determination by the determining section 85b. Also, the present embodiment is configured such that when the operator operates the threshold setting section 90a, the standard threshold value, for example, can be changed and set to a value desired by the operator, so as to be used.

Further, in the control apparatus 66, there is provided a driving force setting section 90b which is used so that the driving force for driving the joystick 62 is adjusted at the time when the information of force acting on the bending section 42 is fed back to the joystick 62 to drive the joystick 62. The driving force setting section 90b outputs a coefficient K as will be described below to the CPU 85. On the basis of the coefficient K, the CPU 85 adjusts the driving force applied to the joystick 62 as the instruction input section.

For example, when, as shown in Fig. 14, the force information Fs is fed back from the bending section 42 and inputted into the CPU 85 of the control apparatus 66 which configures the joystick control section 93, the CPU 85 normally drives the joystick 62 as the instruction input section by driving force Fs' corresponding to the force information Fs.

On the other hand, when the driving force setting section 90b is operated by the operator, the joystick 62 is driven by driving force K·Fs' obtained by multiplying the force information Fs by the coefficient K which can be variably set. Note that the coefficient K is set, for example, as 0 < K < 10.

The operator is able to use the joystick 62 by setting the coefficient K to a value with which the force fed back to the joystick 62 is set to a magnitude to be easily felt by the operator. Note that the threshold setting section 90a and the driving force setting section 90b may be applied to the other embodiment.

Further, an acceleration sensor (not shown) is provided in the control apparatus 66. In the case where an impact of a predetermined value or more is applied to the control apparatus 66, or where an impact of a predetermined value or more is applied to the endoscope 32D, the light source apparatus 37, the video processor 38, the display monitor 39, and the like, which configure the endoscope system 61, the CPU 85 stops the operations of the power sections which actively drive the bending section 42 and the joystick 62, and stops the supply of the electric power to the respective sections.

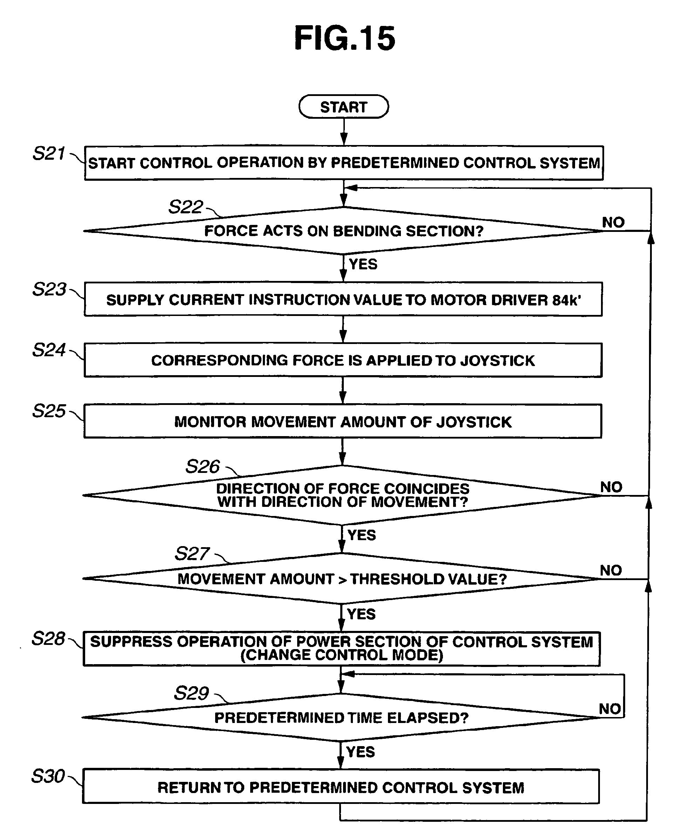

The operation in the present embodiment is realized by the processing procedure as shown in Fig. 15. The contents of the processing procedure shown in Fig. 15 are almost the same as the contents of the processing procedure shown in Fig. 4 in the case where the components in Fig. 15 are replaced by the corresponding components in Fig. 4.

That is, when the power source of the endoscope system 61 in Fig. 13 is turned on, the endoscope system 61 starts the control operation in the predetermined control system, as shown in step S21. Specifically, the CPU 85 starts the control operation based on the control mode of the force reflecting type bilateral control system shown in Fig. 14.

In next step S22, the CPU 85 is set in the state of waiting for detection signals of the tension sensors 83 a to 83d which indicate whether or not a force acts on the bending section 42 (or on the rivet 77 as the joint shaft of the bending section 42).

Then, when the action of the force is detected, the CPU 85 supplies, as shown in step S23, a current command value corresponding to the force to the motor driver 84k' (k = a or b). Here, k corresponds to the detection signal of the tension sensors 81a and 81b. Further, the current command value is set to include the direction of rotation of the motor 81 k'.

Then, as shown in step S24, the force corresponding to the bending direction of the bending section 42 is applied to (the roller 86k of) the joystick 62 by the motor 8 1 k' driven by the motor driver 84k'.

Further, as shown in step S25, the CPU 85 monitors the movement amount (amount of tilting movement) of the joystick 62 on the basis of the detection signal of the encoder 82k'.

Then, as shown in step S26, the CPU 85 determines whether or not the direction of the force applied to the joystick 62 coincides with the direction of the (tilting) movement of the joystick 62 (which is detected in the preceding step S24). When the direction of the force applied to the joystick 62 is not coincident with the direction of the movement of the joystick 62, the CPU 85 returns to step S22.

On the other hand, when it is determined that the direction of the force applied to the joystick 62 coincides with the direction of the movement of the joystick 62, the CPU 85 determines, in next step S27, whether or not the movement amount exceeds the predetermined threshold value.

When the movement amount does not exceed the predetermined threshold value, the CPU 85 returns to step S22. On the contrary, when determining that the movement amount exceeds the predetermined threshold value, the CPU 85 suppresses, in next step S28, the operation of the power section in the control system, or changes the control mode to the other control mode. Note that it may also be configured such that the CPU 85 suppresses the operation of the power section by changing the control parameter.

Specifically, as the processing in step S28, the CPU 85 stops the operation of both the power sections of the motors 81k' and 81k, or stops the operation of at least one of the power sections of the motors 81k' and 81 k. Alternatively, in the case where the control system before the determination is the force reflecting type bilateral control system as shown in Fig. 14, the CPU 85 may change the control mode of the force reflecting type bilateral control system to the control mode of the unilateral control system as shown in Fig. 5. Alternatively, the CPU 85 changes the control parameter.

Further, in this case, the CPU 85 displays the determination result in step S27 or the information in step S28 in the display section of the operation panel 89 and the bending control information display area 39b of the display monitor 39, so as to notify the determination result or the information to the operator.

Further, in next step S29, the CPU 85 is set in the state of waiting for the elapse of the predetermined time on the basis of the timer started by the determination result.

Then, when the predetermined time elapses, the CPU 85 performs, as shown in step S30, the processing to return the control state to the state of the first control system, and returns to step S22.

According to the present embodiment, in such a case where the operator grasps the joystick 62 and performs a bending instruction operation (bending input operation) of the bending section 42, it is possible to effectively prevent an operation which is not intended by the operator.

Further, it is possible that the control state is returned to the normal control state after the predetermined time, and thereby an endoscope examination, treatment, and the like, can be continued in a state of good operability.

Note that the case of the endoscope 32D which has the bending section 42 and which functions as an active medical apparatus is described as embodiment 3, but embodiment 3 can be applied to the case other than the case of the endoscope 32D.

Specifically, embodiment 3 can be similarly applied in the case where an active overtube is formed by providing, as shown in Fig. 13, the electrically driven bending section 42 in a thin and long insertion auxiliary member or insertion guide member (so-called overtube), which has a channel in which one of the treatment instruments 1B to 1C can be inserted, or a channel in which the insertion section 33 of the endoscopes 32 and 32D can be inserted, and where the bending section 42 is electrically driven and controlled as described in embodiment 3.

Note that an embodiment, and the like, configured by partially combining the above described embodiments is also included within the scope of the present invention.

Further, it may also be configured such that in such a case where a plurality of kinds of medical apparatuses are connected to the control apparatus 66, optimum control parameters are automatically set or selected according to a combination and kinds of the medical apparatuses connected to the control apparatus 66.

## Claims

1. An active medical apparatus system (1) comprising:
an active medical apparatus (4) including at least one rotatable joint;
an active medical apparatus driving section (5) which electrically drives the active medical apparatus (4);
an instruction input section (2) with which an operator performs instruction input to drive the active medical apparatus (4);
an instruction input section driving section (3) to which information of a force acting on the active medical apparatus (4) is fed back and inputted, and which electrically drives the instruction input section (2) according to the information of the force, **characterized by** the active medical apparatus system (1) further comprising:
a determining section (21a) which determines whether or not a movement of the instruction input section (2) in an acting direction of a driving force driving the instruction input section (2) is generated in an amount of a predetermined threshold value or more in a state where the information of the force acting on the active medical apparatus (4) is fed back and inputted to the instruction input section (2); and
a control section (6) which, in a case where it is determined by the determining section (21a) that the movement of the instruction input section (2) is generated in the amount of the predetermined threshold value or more, suppresses a driving of at least one of the instruction input section driving section (3) and the active medical apparatus driving section (5).

2. The active medical apparatus system (1) according to claim 1, further comprising:
a bending section (42) formed by including a plurality of the joints; and
an electric bending endoscope or an active overtube, each of which electrically drives and bends the bending section (42), or an active treatment instrument which electrically curves or bends the joints.

3. The active medical apparatus system (1) according to claim 1, wherein the instruction input section (2) is a tiltably movable joystick (58).

4. The active medical apparatus system (1) according to claim 1, wherein the instruction input section (2) has a shape substantially similar to a shape of the active medical apparatus (4).

5. The active medical apparatus system (1) according to claim 1, wherein in a case where the determination result is obtained by the determining section (21a), the control section (6) changes a control mode by which the driving of the instruction input section driving section (3) and of the active medical apparatus driving section (5) is controlled, from the control mode before the determination result.

6. The active medical apparatus system (1) according to claim 2, wherein in a case where the determination result is obtained by the determining section (21a), the control section (6) changes a control mode, by which the driving of the instruction input section driving section (3) and of the active medical apparatus driving section (5) is controlled, from the control mode before the determination result.

7. The active medical apparatus system (1) according to claim 5, wherein in a case where the determination result is obtained by the determining section (21a), the control section (6) changes from the control mode in which the force information before the determination result is fed back, to the control mode in which the force information is not fed back after the determination result.

8. The active medical apparatus system (1) according to claim 7, wherein the control mode of feeding back the force information is a force reflecting type bilateral control mode or a force feedback type bilateral control mode.

9. The active medical apparatus system (1) according to claim 1, wherein in a case where the determination result is obtained by the determining section (21a), the control section (6) changes a control parameter used for driving the instruction input section driving section (3) and the active medical apparatus driving section (5).

10. The active medical apparatus system (1) according to claim 2, wherein in a case where the determination result is obtained by the determining section (21a), the control section (6) changes a control parameter which is used for driving the instruction input section (2) by the instruction input section driving section (3), or a control parameter which is used for driving the active medical apparatus (4) by the active medical apparatus driving section (5).

11. The active medical apparatus system (1) according to claim 10, wherein in a case where the determination result is obtained by the determining section (21a), the control section (6) changes the control parameter so as to suppress the driving of at least one of the instruction input section (2) and the active medical apparatus (4).

12. The active medical apparatus system (1) according to claim 1, wherein the control section (6) further cancels the suppression after the elapse of a predetermined time from when the determination result is obtained by the determining section (21a).

13. The active medical apparatus system (1) according to claim 1, further comprising a presentation section which presents information determined by the determining section (21a).

14. The active medical apparatus system (1) according to claim 1, further comprising a display section which in a case where the control mode is changed, displays the changed control mode.

15. The active medical apparatus system (1) according to claim 1,
wherein the active medical apparatus (4) is configured by using a first joint and a second joint as the joint,
wherein the instruction input section (2) has a first instruction input section and a second instruction input section which respectively drive the first joint and the second joint, and
wherein the first instruction input section and the second instruction input section are respectively driven by a first driving force and a second driving force which respectively correspond to information of forces respectively applied to the first joint and the second joint by the instruction input section driving section (3).

16. The active medical apparatus system (1) according to claim 15,
wherein the determining section (21a) respectively determines whether or not the movement of the first instruction input section in the acting direction of the first driving force acting on the first instruction input section is generated in an amount of a predetermined threshold value or more, and
whether or not the movement of the second instruction input section in the acting direction of the second driving force acting on the second instruction input section is generated in an amount of a predetermined threshold value or more.

17. The active medical apparatus system (1) according to claim 1, further comprising:
an electric bending endoscope (32D) which electrically drives and bends a bending section (42) formed by including a plurality of joints; and
a signal processing apparatus which performs signal processing to an image picked up by an image pickup device provided in the electric bending endoscope (32D).

18. The active medical apparatus system (1) according to claim 17, further comprising a display monitor (39) which displays an endoscopic image generated by the signal processing by the signal processing apparatus, wherein the display monitor (39) presents information determined by the determining section (21a).

19. The active medical apparatus system (1) according to claim 1, further comprising a driving force setting section (90b) which variably sets, on the basis of the force information, a magnitude of the driving force for electrically driving the instruction input section (2).

20. The active medical apparatus system (1) according to claim 1, further comprising a threshold setting section (90a) which variably sets the threshold value.

## Patentansprüche

1. Aktives medizinisches Vorrichtungssystem (1) mit:
einer aktiven medizinischen Vorrichtung (4), die mindestens ein Drehgelenk aufweist;
einem Antriebsabschnitt (5) der aktiven medizinischen Vorrichtung, der die aktive medizinische Vorrichtung (4) elektrisch antreibt;
einem Befehlseingabeabschnitt (2), mit dem eine Bedienungsperson eine Befehlseingabe vornimmt, um die aktive medizinische Vorrichtung (4) anzutreiben;
einem Befehlseingabeabschnitt-Antriebsabschnitt (3), an den Information über eine auf die aktive medizinische Vorrichtung (4) einwirkende Kraft rückgekoppelt und eingegeben wird, und der den Befehlseingabeabschnitt (2) gemäß der Information über die Kraft elektrisch antreibt, **dadurch gekennzeichnet, dass** das aktive medizinische Vorrichtungssystem (1) ferner aufweist:
einen Bestimmungsabschnitt (21a), der bestimmt, ob eine Bewegung des Befehlseingabeabschnitts (2) in einer Wirkrichtung einer den Befehlseingabeabschnitt (2) antreibenden Antriebskraft in einem Umfang eines vorbestimmten Schwellenwerts oder mehr in einem Zustand erzeugt wird oder nicht, in dem die Information über die auf die aktive medizinische Vorrichtung (4) einwirkende Kraft rückgekoppelt und in den Befehlseingabeabschnitt (2) eingegeben wird; und
einen Steuerabschnitt (6), der in einem Fall, in dem von dem Bestimmungsabschnitt (21a) bestimmt wird, dass die Bewegung des Befehlseingabeabschnitts (2) in dem Umfang des vorbestimmten Schwellenwerts oder mehr erzeugt wird, einen Antrieb des Befehlseingabeabschnitt-Antriebsabschnitts (3) und/oder des Antriebsabschnitts (5) der aktiven medizinischen Vorrichtung abstellt.

2. Aktives medizinisches Vorrichtungssystem (1) nach Anspruch 1, ferner mit:
einem Biegeabschnitt (42), der durch Aufnahme mehrerer der Gelenke gebildet ist; und
einem elektrischen Biegeendoskop oder einem aktiven Übertubus, von denen jedes/jeder den Biegeabschnitt (42) elektrisch antreibt und biegt, oder einem aktiven Behandlungsinstrument, das die Gelenke elektrisch krümmt oder biegt.

3. Aktives medizinisches Vorrichtungssystem (1) nach Anspruch 1, wobei der Befehlseingabeabschnitt (2) ein schwenkbar beweglicher Joystick (58) ist.

4. Aktives medizinisches Vorrichtungssystem (1) nach Anspruch 1, wobei der Befehlseingabeabschnitt (2) eine Form aufweist, die im Wesentlichen ähnlich einer Form der aktiven medizinischen Vorrichtung (4) ist.

5. Aktives medizinisches Vorrichtungssystem (1) nach Anspruch 1, wobei in einem Fall, in dem das Bestimmungsergebnis von dem Bestimmungsabschnitt (21a) erhalten wird, der Steuerabschnitt (6) einen Steuermodus, durch den der Antrieb des Befehlseingabeabschnitt-Antriebsabschnitts (3) und des Antriebsabschnitts (5) der aktiven medizinischen Vorrichtung gesteuert wird, gegenüber dem Steuermodus vor dem Bestimmungsergebnis ändert.

6. Aktives medizinisches Vorrichtungssystem (1) nach Anspruch 2, wobei in einem Fall, in dem das Bestimmungsergebnis von dem Bestimmungsabschnitt (21a) erhalten wird, der Steuerabschnitt (6) einen Steuermodus, durch den der Antrieb des Befehlseingabeabschnitt-Antriebsabschnitts (3) und des Antriebsabschnitts (5) der aktiven medizinischen Vorrichtung gesteuert wird, gegenüber dem Steuermodus vor dem Bestimmungsergebnis ändert.

7. Aktives medizinisches Vorrichtungssystem (1) nach Anspruch 5, wobei in einem Fall, in dem das Bestimmungsergebnis von dem Bestimmungsabschnitt (21a) erhalten wird, der Steuerabschnitt (6) von dem Steuermodus, bei dem die Kraftinformation vor dem Bestimmungsergebnis rückgekoppelt wird, zu dem Steuermodus wechselt, bei dem die Kraftinformation nach dem Bestimmungsergebnis nicht rückgekoppelt wird.

8. Aktives medizinisches Vorrichtungssystem (1) nach Anspruch 7, wobei der Steuermodus des Rückkoppelns der Kraftinformation ein bilateraler Steuermodus vom Kraftreflexionstyp oder ein bilateraler Steuermodus vom Kraftrückkoppelungstyp ist.

9. Aktives medizinisches Vorrichtungssystem (1) nach Anspruch 1, wobei in einem Fall, in dem das Bestimmungsergebnis von dem Bestimmungsabschnitt (21a) erhalten wird, der Steuerabschnitt (6) einen Steuerparameter ändert, der zum Antrieb des Befehlseingabeabschnitt-Antriebsabschnitts (3) und des Antriebsabschnitts (5) der aktiven medizinischen Vorrichtung verwendet wird.

10. Aktives medizinisches Vorrichtungssystem (1) nach Anspruch 2, wobei in einem Fall, in dem das Bestimmungsergebnis von dem Bestimmungsabschnitt (21a) erhalten wird, der Steuerabschnitt (6) einen Steuerparameter ändert, der zum Antrieb des Befehlseingabeabschnitts (2) durch den Befehlseingabeabschnitt-Antriebsabschnitt (3) verwendet wird, oder einen Steuerparameter, der zum Antrieb der aktiven medizinischen Vorrichtung (4) durch den Antriebsabschnitt (5) der aktiven medizinischen Vorrichtung verwendet wird.

11. Aktives medizinisches Vorrichtungssystem (1) nach Anspruch 10, wobei in einem Fall, in dem das Bestimmungsergebnis von dem Bestimmungsabschnitt (21a) erhalten wird, der Steuerabschnitt (6) den Steuerparameter so ändert, dass der Antrieb des Befehlseingabeabschnitts (2) und/oder der aktiven medizinischen Vorrichtung (4) abgestellt wird.

12. Aktives medizinisches Vorrichtungssystem (1) nach Anspruch 1, wobei der Steuerabschnitt (6) ferner das Abstellen nach dem Verstreichen einer vorbestimmten Zeit seit dem Erhalt des Bestimmungsergebnisses durch den Bestimmungsabschnitt (21a) aufhebt.

13. Aktives medizinisches Vorrichtungssystem (1) nach Anspruch 1, ferner mit einem Präsentationsabschnitt, der von dem Bestimmungsabschnitt (21a) bestimmte Information präsentiert.

14. Aktives medizinisches Vorrichtungssystem (1) nach Anspruch 1, ferner mit einem Anzeigeabschnitt, der in einem Fall, in dem der Steuermodus geändert wird, den geänderten Steuermodus anzeigt.

15. Aktives medizinisches Vorrichtungssystem (1) nach Anspruch 1,
wobei die aktive medizinische Vorrichtung (4) durch Anwenden eines ersten Gelenks und eines zweiten Gelenks als das Gelenk konfiguriert ist,
wobei der Befehlseingabeabschnitt (2) einen ersten Befehlseingabeabschnitt und einen zweiten Befehlseingabeabschnitt aufweist, die jeweils das erste Gelenk und das zweite Gelenk antreiben, und
wobei der erste Befehlseingabeabschnitt und der zweite Befehlseingabeabschnitt jeweils von einer ersten Antriebskraft und einer zweiten Antriebskraft angetrieben werden, die jeweils Information über Kräfte entsprechen, welche von dem Befehlseingabeabschnitt-Antriebsabschnitt (3) auf das erste Gelenk und das zweite Gelenk aufgebracht werden.

16. Aktives medizinisches Vorrichtungssystem (1) nach Anspruch 15,
wobei der Bestimmungsabschnitt (21a) jeweils bestimmt, ob die Bewegung des ersten Befehlseingabeabschnitts in der Wirkrichtung der auf den ersten Befehlseingabeabschnitt einwirkenden ersten Antriebskraft in einem Umfang eines vorbestimmten Schwellenwerts oder mehr erzeugt wird oder nicht, und
ob die Bewegung des zweiten Befehlseingabeabschnitts in der Wirkrichtung der auf den zweiten Befehlseingabeabschnitt einwirkenden zweiten Antriebskraft in einem Umfang eines vorbestimmten Schwellenwerts oder mehr erzeugt wird oder nicht.

17. Aktives medizinisches Vorrichtungssystem (1) nach Anspruch 1, ferner mit:
einem elektrischen Biegeendoskop (32D), das einen Biegeabschnitt (42), der durch Aufnahme mehrerer Gelenke gebildet ist, elektrisch antreibt und biegt; und
einer Signalverarbeitungsvorrichtung, die eine Signalverarbeitung an einem Bild durchführt, das von einer in dem elektrischen Biegeendoskop (32D) vorgesehenen Bildaufnahmevorrichtung aufgenommen wird.

18. Aktives medizinisches Vorrichtungssystem (1) nach Anspruch 17, ferner mit einem Anzeigemonitor (39), der ein von der Signalverarbeitung durch die Signalverarbeitungsvorrichtung erzeugtes endoskopisches Bild anzeigt, wobei der Anzeigemonitor (39) von dem Bestimmungsabschnitt (21a) bestimmte Information präsentiert.

19. Aktives medizinisches Vorrichtungssystem (1) nach Anspruch 1, ferner mit einem Antriebskraft-Einstellabschnitt (90b), der auf der Basis der Kraftinformation eine Größe der Antriebskraft zum elektrischen Antrieb des Befehlseingabeabschnitts (2) variabel einstellt.

20. Aktives medizinisches Vorrichtungssystem (1) nach Anspruch 1, ferner mit einem Schwellen-Einstellabschnitt (90a), der den Schwellenwert variabel einstellt.

## Revendications

1. Système d'appareil médical actif (1) comprenant :
un appareil médical actif (4) incluant au moins une articulation rotative ;
une section d'entraînement d'appareil médical actif (5) qui entraîne électriquement l'appareil médical actif (4) ;
une section d'entrée d'instruction (2) avec laquelle un opérateur exécute une entrée d'instruction pour entraîner l'appareil médical actif (4) ;
une section d'entraînement de section d'entrée d'instruction (3) à laquelle une information d'une force agissant sur l'appareil médical actif (4) est renvoyée et entrée, et qui entraîne électriquement la section d'entrée d'instruction (2) en fonction de l'information de la force, **caractérisé par** le système d'appareil médical actif (1) comprenant en outre :
une section de détermination (21a) qui détermine si un mouvement de la section d'entrée d'instruction (2) dans une direction d'action d'une force d'entraînement entraînant la section d'entrée d'instruction (2) est généré ou non dans une quantité d'une valeur de seuil prédéterminée ou plus dans un état où l'information de la force agissant sur l'appareil médical actif (4) est renvoyée et entrée dans la section d'entrée d'instruction (2) ; et
une section de commande (6) qui, dans un cas où il est déterminé par la section de détermination (21a) que le mouvement de la section d'entrée d'instruction (2) est généré dans la quantité de la valeur de seuil prédéterminée ou plus, supprime un entraînement d'au moins une de la section d'entraînement de section d'entrée d'instruction (3) et de la section d'entraînement d'appareil médical actif (5).

2. Système d'appareil médical actif (1) selon la revendication 1, comprenant en outre :
une section de flexion (42) formée en incluant une pluralité des articulations ; et
un endoscope électrique à flexion ou un sur-tube actif, chacun desquels entraîne et fait fléchir électriquement la section de flexion (42), ou un instrument de traitement actif qui courbe ou fait fléchir électriquement les articulations.

3. Système d'appareil médical actif (1) selon la revendication 1, dans lequel la section d'entrée d'instruction (2) est une manette (58) mobile en inclinaison.

4. Système d'appareil médical actif (1) selon la revendication 1, dans lequel la section d'entrée d'instruction (2) a une forme sensiblement similaire à une forme de l'appareil médical actif (4).

5. Système d'appareil médical actif (1) selon la revendication 1, dans lequel, dans un cas où le résultat de détermination est obtenu par la section de détermination (21a), la section de commande (6) change un mode de commande par lequel l'entraînement de la section d'entraînement de section d'entrée d'instruction (3) et de la section d'entraînement d'appareil médical actif (5) est commandé, par rapport au mode de commande avant le résultat de détermination.

6. Système d'appareil médical actif (1) selon la revendication 2, dans lequel, dans un cas où le résultat de détermination est obtenu par la section de détermination (21a), la section de commande (6) change un mode de commande par lequel l'entraînement de la section d'entraînement de section d'entrée d'instruction (3) et de la section d'entraînement d'appareil médical actif (5) est commandé, par rapport au mode de commande avant le résultat de détermination.

7. Système d'appareil médical actif (1) selon la revendication 5, dans lequel, dans un cas où le résultat de détermination est obtenu par la section de détermination (21a), la section de commande (6) passe du mode de commande dans lequel l'information de force avant le résultat de détermination est renvoyée, au mode de commande dans lequel l'information de force n'est pas renvoyée après le résultat de détermination.

8. Système d'appareil médical actif (1) selon la revendication 7, dans lequel le mode de commande consistant à renvoyer l'information de force est un mode de commande bilatéral de type à réflexion de force ou un mode de commande bilatéral de type à renvoi de force.

9. Système d'appareil médical actif (1) selon la revendication 1, dans lequel, dans un cas où le résultat de détermination est obtenu par la section de détermination (21a), la section de commande (6) change un paramètre de commande utilisé pour entraîner la section d'entraînement de section d'entrée d'instruction (3) et la section d'entraînement d'appareil médical actif (5).

10. Système d'appareil médical actif (1) selon la revendication 2, dans lequel, dans un cas où le résultat de détermination est obtenu par la section de détermination (21a), la section de commande (6) change un paramètre de commande qui est utilisé pour entraîner la section d'entrée d'instruction (2) par la section d'entraînement de section d'entrée d'instruction (3), ou un paramètre de commande qui est utilisé pour entraîner l'appareil médical actif (4) par la section d'entraînement d'appareil médical actif (5).

11. Système d'appareil médical actif (1) selon la revendication 10, dans lequel, dans un cas où le résultat de détermination est obtenu par la section de détermination (21a), la section de commande (6) change le paramètre de commande de manière à supprimer l'entraînement d'au moins un de la section d'entrée d'instruction (2) et de l'appareil médical actif (4).

12. Système d'appareil médical actif (1) selon la revendication 1, dans lequel la section de commande (6) annule en outre la suppression après l'écoulement d'une durée prédéterminée commençant quand le résultat de détermination est obtenu par la section de détermination (21a).

13. Système d'appareil médical actif (1) selon la revendication 1, comprenant en outre une section de présentation qui présente une information déterminée par la section de détermination (21a).

14. Système d'appareil médical actif (1) selon la revendication 1, comprenant en outre une section d'affichage qui, dans un cas où le mode de commande est changé, affiche le mode de commande changé.

15. Système d'appareil médical actif (1) selon la revendication 1,
dans lequel l'appareil médical actif (4) est configuré en utilisant une première articulation et une deuxième articulation comme l'articulation,
dans lequel la section d'entrée d'instruction (2) a une première section d'entrée d'instruction et une deuxième section d'entrée d'instruction qui entraînent respectivement la première articulation et la deuxième articulation, et
dans lequel la première section d'entrée d'instruction et la deuxième section d'entrée d'instruction sont respectivement entraînées par une première force d'entraînement et une deuxième force d'entraînement qui correspondent respectivement à des informations de forces respectivement appliquées à la première articulation et la deuxième articulation par la section d'entraînement de section d'entrée d'instruction (3).

16. Système d'appareil médical actif (1) selon la revendication 15,
dans lequel la section de détermination (21a) détermine respectivement si le mouvement de la première section d'entrée d'instruction dans la direction d'action de la première force d'entraînement agissant sur la première section d'entrée d'instruction est ou non généré dans une quantité d'une valeur de seuil prédéterminée ou plus, et
si le mouvement de la deuxième section d'entrée d'instruction dans la direction d'action de la deuxième force d'entraînement agissant sur la deuxième section d'entrée d'instruction est ou non généré dans une quantité d'une valeur de seuil prédéterminée ou plus.

17. Système d'appareil médical actif (1) selon la revendication 1, comprenant en outre :
un endoscope électrique à flexion (32D) qui entraîne et courbe électriquement une section de flexion (42) formée en incluant une pluralité d'articulations ; et
un appareil de traitement de signaux qui exécute un traitement de signaux sur une image capturée par un dispositif de capture d'images prévu dans l'endoscope électrique à flexion (32D).

18. Système d'appareil médical actif (1) selon la revendication 17, comprenant en outre un moniteur d'affichage (39) qui affiche une image endoscopique générée par le traitement de signaux par l'appareil de traitement de signaux, dans lequel le moniteur d'affichage (39) présente une information déterminée par la section de détermination (21a).

19. Système d'appareil médical actif (1) selon la revendication 1, comprenant en outre une section de fixation de force d'entraînement (90b) qui fixe de manière variable, sur la base de l'information de force, une grandeur de la force d'entraînement pour entraîner électriquement la section d'entrée d'instruction (2).

20. Système d'appareil médical actif (1) selon la revendication 1, comprenant en outre une section de fixation de seuil (90a) qui fixe de manière variable la valeur de seuil.
